# EUROPEAN PATENT APPLICATION

(11) **EP 4 356 941 A1**
(43) Date of publication of application: **24.04.2024**
(21) Application number: 22202517.3
(22) Date of filing: 19.10.2022
(51) Int. Cl.: A61M 3/02, A61M 25/00, A61M 25/04

(54) **RECTAL CATHETER WITH FLUID RESERVOIR AND METHOD FOR MANUFACTURING SAID RECTAL CATHETER**

(71) Applicant: Wellspect AB, 431 21 Mölndal (SE)
(72) Inventor: Pedersen, Lars, 431 39 Mölndal (SE); Börstell, Thomas, 415 73 Göteborg (SE)
(74) Representative: Lind Edlund Kenamets Intellectual Property AB

(57) **Abstract**

The present disclosure relates to a catheter (1) and a method for manufacturing the catheter (1). The catheter (1) comprises a core tube (10) with a lumen (15) extending from a rear end to a front end along an elongation axis (x), a front portion (13) and a rear portion (11). The catheter (1) further comprises a flexible outer layer (20) forming a fluid reservoir (25, 25') containing a fluid between the flexible outer layer (20) and at least a part of the front portion (13). The flexible outer layer (20) optionally comprises one or more radial eyelet(s) (23) arranged in front of the front portion (13) of the core tube (10) and being in fluid communication with the front opening (14), wherein the flexible outer layer (20) is more flexible than the core tube (10). Optionally, the catheter (1) further comprises a flexible inner layer (20').

## Description

### TECHNICAL FIELD OF THE INVENTION

The present invention relates to a catheter suitable for rectal irrigation, and a method for manufacturing the catheter.

### BACKGROUND OF THE INVENTION

A catheter is a medical instrument useable for many types of uses and applications, comprising an elongated body through which one or more channels/lumens extends. For instance, catheters may be used to perform rectal irrigation, such as Trans Anal Irrigation (TAI), which involves introducing a catheter into the anal canal of a patient and injecting a fluid (e.g. water) to flush out stool and stimulate the rectum and bowels of the patient. Rectal catheters used for this purpose are often provided with a retention element, which may function to retain the catheter in an inserted position, and/or to form a seal against the anal canal when inserted into the rectum of a user. The retention element may e.g. be in the form of a rigid cone which surrounds the elongated body and seals the anal canal or rectum when the catheter is used to inject fluid.

Alternatively, a rectal catheter may be provided with an inflatable element (e.g. a balloon element) which surrounds the elongated body, and which is inflated inside the anal canal to create a seal and/or to retain the catheter in the anal canal. To this end, rectal catheters may feature at least two separate channels, a first channel for the injection of irrigation fluids and a second channel which at a first end is terminated inside the inflatable element for inflation of the inflatable element. By injecting a fluid, such as air or water, in the channel of the inflatable element the level of inflation may be controlled such that the inflatable element creates a seal and retains the catheter in the inserted position. When the catheter is to be removed, the same channel may be used for removing fluid from the inflatable element so as to deflate the inflatable element.

A problem with prior solutions is that it may be complicated to inflate a retention element sufficiently and correctly so as to form a tight seal. For instance, many solutions are adapted to allow a patient to perform anal irrigation without any aid from a second person. To enable this type of self-treatment, electrically powered pump systems have been proposed which connect to a rectal catheter and allows the patient to individually control the flow of air into the inflatable retention element and the flow of water for irrigation at the press of a button. Additionally, manually powered pump systems have also been proposed allowing the user to operate a pump (e.g. by actuating a pump handle or squeezing a fluid reservoir) to inflate the retention element and inject irrigation fluid. However, such systems tend to be bulky and inconvenient to transport while also being complex and expensive to manufacture or repair since they necessitate two separate pump systems (one for irrigation using water and one for inflation of the retention element using e.g. air) and/or an intricate arrangement of actuated valves to switching between pumping a fluid into the first and second channel of the catheter.

Other drawbacks with existing solutions are that there is a risk of the inflatable retention element bursting during inflation and that it is difficult for users to ensure that the balloon is sufficiently inflated to create a proper seal before the user injects the irrigation fluid.

### SUMMARY OF THE INVENTION

In view of the drawbacks of existing solutions there is a need for an improved catheter for rectal irrigation which is easy to use and manufacture while still overcoming at least some of the drawbacks mentioned in the above. It is a purpose of the present invention to provide such an improved catheter and a method for manufacturing the catheter.

According to a first aspect of the invention there is provided a catheter comprising: a core tube extending along an elongation axis, the core tube comprising a lumen extending through the core tube and between a front opening in a front portion of the core tube and a rear opening in a rear portion opposite the front portion. The catheter further comprises a flexible outer layer disposed around the front portion, the flexible outer layer covering the front portion along the elongation axis and at least partially covering the front portion in a radial direction. The flexible outer layer forming a fluid reservoir containing a fluid between the flexible outer layer and at least a part of the front portion, such that the flexible outer layer and the fluid reservoir forms a resiliently deformable body. The flexible outer layer further comprises at least one radial eyelet arranged in front of the front portion of the core tube wherein the at least one radial eyelet is in fluid communication with the front opening. Additionally, the flexible outer layer of the catheter is more flexible than the core tube.

It is noted that it is entirely optional for the flexible outer layer to cover the core tube along the elongation axis and comprise a radial eyelet arranged in front of the front opening. While a catheter with these features exhibits some beneficial properties it is envisaged that the catheter not necessarily comprises these features and alternatively comprises two flexible layers (one outer flexible layer and one inner flexible layer) as will be described in connection to the second aspect of the invention in the below.

In some implementations, the catheter is a rectal catheter such as a rectal irrigation catheter. It is however envisaged that the catheter also could be configured to be used as a urinary catheter, dilation catheter, venous catheter, and the like.

While the flexible outer layer is more flexible than the core tube it is understood that the core tube may also exhibit some degree of flexibility so to allow the core tube to bend or twist when introduced through a patient's anus. This makes insertion of the catheter more comfortable and mitigates the risk of damaging the patients rectal canal or bowel.

The front portion is the portion of the core tube which is first introduced into the anal canal of a patient with the rear portion following behind the insertion portion. Accordingly, an elongation axis may be defined extending from a rear end of the catheter towards an insertion front end of the core tube. Similarly, the flexible outer layer which covers the core tube also extends along the elongation axis from a rear end to a front insertion end. As, according to this first aspect, the flexible outer layer covers the outside of the front portion of the core tube along the elongation axis, the front insertion end of the flexible outer layer is located in front of the front insertion end of the core tube. That is, the flexible outer layer wraps around the front insertion portion of the core tube. In some examples described in the below, the front insertion end of the flexible outer layer extends beyond the core tube in the forward direction. Alternatively, the core tube comprises a scaffolding supporting the flexible outer layer from the inside.

The core tube is less flexible than the flexible outer layer which is flexible and/or elastic. The core tube and the flexible outer layer may be made of different materials. For example, the core tube could be made of materials such as polyurethane, polyolefin, polyethylene, or combinations thereof. The flexible outer layer can e.g. be made of silicone or elastic polymers. The fluid in the fluid reservoir may be a gel, mineral oil, glycerin or the like. Alternatively, the fluid in the fluid reservoir is a liquid (such as water) or a gas (such as air or nitrogen). Preferably, the fluid is a non-compressible fluid which ensures that the pressure is maintained low in the unlikely scenario wherein the fluid reservoir bursts. It is also preferred that the fluid in the fluid reservoir is of a sufficiently high viscosity which slows the redistribution of the fluid inside the reservoir and dampens any sudden changes in shape.

Since the core tube is less flexible than the flexible outer layer, it enables insertion of the catheter without the need of any additional insertion aid. It also ensures that the lumen of the catheter is maintained open and unrestricted during use.

With a fluid reservoir it is meant a closed volume, the closed volume being preferably annulus in shape, containing a fluid which is confined only in the fluid reservoir. If the fluid reservoir is subject to an external pressure, its outer shape changes due to the fluid being rearranged inside the reservoir without any fluid being removed or added to the fluid reservoir. Thus, the fluid reservoir is configured to be inserted into, seal, and be removed from the anal canal without introduction/removal of fluid to/from the fluid reservoir. Accordingly, a fluid reservoir is not to be confused with an inflatable reservoir or balloon used for other types of catheters. With such an inflatable reservoir or balloon, fluid must be added after inserting the catheter into the anus to create the seal and then the fluid must be removed before being able to remove the catheter from the anal canal, which is not the case for the permanently sealed fluid reservoir formed between the core tube and the flexible outer layer described in the above.

The fluid reservoir and the flexible outer layer forms a resiliently deformable body which surrounds at least a portion of the front portion of the core tube. The resiliently deformable body is filled with sufficient fluid so as to exert a pressure onto the flexible outer layer and stretch the flexible outer layer to assume a predetermined shape. The resiliently deformable body may then be deformed, e.g. by an external force acting on the resiliently deformable body, so that fluid is relocated to other parts of the fluid reservoir. When the external force is removed the resiliently deformable body reverts to its initial predetermined shape. The predetermined shape may e.g. be dictated by the shape of the flexible outer layer when it was cured during its manufacturing. For example, the predetermined shape may be dictated by the shape of a mandrel used to form the flexible outer layer in a dipping process, the shape of the mold used to mold the flexible outer layer, the shape into which the flexible outer layer has been thermoformed or by process parameters and constituents/chemicals of the dipping process.

In some implementations, the flexible outer layer comprises two or more radial eyelets which are angularly separated around the elongation axis and/or linearly separated along the elongation axis.

According to a second aspect of the invention there is provided a catheter (such as a rectal catheter for rectal irrigation) comprising a core tube extending from a rear end to a front end along an elongation axis, the core tube comprising a lumen extending between a front opening in a front portion and a rear opening in a rear portion, opposite said front portion. The catheter further comprises a flexible outer layer disposed around the front portion, the flexible outer layer at least partially covering the front portion in a radial direction and a flexible inner layer disposed between the flexible outer layer and the core tube. Wherein the flexible outer layer and the flexible inner layer forms a fluid reservoir containing a fluid between the flexible outer layer and the flexible inner layer, such that the flexible outer layer and the fluid reservoir forms a resiliently deformable body covering at least a part of the front portion of the core tube, and wherein the flexible outer layer and flexible inner layer are more flexible than the core tube.

The flexible inner layer and flexible outer layer may collectively be referred to as an outer sleeve, which in some cases is made as a single piece. However, it is not necessary for the catheter to comprise two flexible layers and, as described in connection to the first aspect of the invention in the above, it is envisaged that the catheter comprises a flexible outer layer which covers the core tube along the elongation axis. Additionally, it is noted that the invention according to the first and second aspect can be combined to form many different embodiments falling within the scope of the first and second aspect. For example, it is envisaged that the catheter may comprise both a flexible inner layer and a flexible outer layer as well as at least one of these covering the core tube in the elongation axis.

The invention is at least partially based on the realization that with a catheter comprising a fluid reservoir, a reliable seal against the medial sides and the cranial side of the anal canal is created automatically upon insertion of the catheter through the anus of a patient, without the need to pump and/or inject an inflation fluid. Accordingly, the catheter enables more convenient use and does not require intricate pump systems for supplying both irrigation fluid and inflation fluid.

Another benefit is that the fluid reservoir may be configured to seal against the inside of the rectal canal in addition to sealing against the rectal canal opening into the rectum. This enables the catheter to be made shorter (compared e.g. to regular balloon catheters) while still enabling a proper seal which increases the safety for the patient.

Yet another benefit of the first aspect of the invention is that since the flexible outer layer covers the core tube along the elongation axis, introduction of the catheter into the anus is more comfortable. Additionally, the flexible outer layer covering the core tube along the elongation axis ensures that the flexible outer layer will not slide off the core tube. At the same time, while the flexible outer layer is prevented from sliding off, the flexibility of the flexible outer layer allows the flexible outer layer to stretch (e.g. elastically) in the rearward direction.

An additional benefit of the second aspect of the invention is that with two flexible layers the outer sleeve can be made separately, and even filled with the fluid, prior to being arranged on the core tube. For example, the outer sleeve may be intended for single use, or intended to be used a finite number of times, and provided separately from the core tube allowing the user to install and remove the outer sleeve from the core tube prior to/after performing rectal irrigation treatment.

In some implementations, the core tube further comprises a tip portion arranged at the front end of the front portion, wherein the tip portion supports the flexible outer layer along the elongation axis.

In some aspects, the tip portion acts as a scaffolding supporting the flexible outer layer in at least the elongation axis while allowing irrigation fluid to pass from the front opening in the core tube to the radial eyelet in the flexible outer layer. For example, the tip portion ensures that during use, the flexible outer layer remains extended and does not slack, kink, or be in other ways deformed, along the elongation axis so that the at least one radial eyelet is held open even when no irrigation is passed through the catheter. The tip portion is preferably formed in a single piece with the core tube.

In some implementations, the tip portion comprises one or more radial eyelet(s) in fluid communication with the lumen and one or more radial eyelet(s) of the flexible outer layer.

That is, the tip portion may be a tubular extension of the front portion wherein the tubular extension comprises a radial eyelet aligned with the radial outlet of the flexible outer layer. Optionally, the tubular extension is terminated at its front end with a cap closing the tip portion along elongation axis. Preferably, the cap is rounded or dome shaped. Additionally or alternatively, the cap is of sufficient diameter to not exercise an extensive point pressure on the rectal wall of the patient.

In some implementations, the flexible outer layer is attached to at least a part of the outside of the rear portion.

The attachment of the flexible outer layer may comprise the flexible outer layer being attached to the rear portion via friction. Additionally, or alternatively, the flexible outer layer is attached by means of heat welding and/or with an adhesive applied between the outside of the rear portion and the flexible outer layer.

In some implementations, the part of the outside of the rear portion to which the flexible outer layer is attached is provided with at least one radial protrusion configured to hold the flexible outer layer in place by means of mechanical support and friction.

That is, the flexible outer layer may be attached using friction, as a friction fit, by being fitted tightly over the at least one radial protrusion. In some implementations, the part of the outside of the rear portion to which the flexible outer layer is attached is provided with at least two radial protrusions configured to hold the flexible outer layer in place using friction wherein the at least two radial protrusions are separated along the elongation axis with an indentation. By pulling the flexible outer layer over the radial protrusions, the flexible outer layer will be reliably attached to the core tube e.g. as the flexible outer layer may be fitted sufficiently tightly so as to penetrate into the indentation between the two radial protrusions.

In some implementations, the rear portion further comprises a connector for connecting the rear opening to an external device.

With a connector in the rear portion, the catheter can easily be removably connected to an external device. Preferably, the connector is placed in the part of the rear portion which is configured to remain outside of the anal canal when the catheter has been inserted into the anus of the patient. In this way, the connector, or the external device connected to the connector, does not come into contact with the patient. In some implementations, the flexible outer layer extends also over the rear portion. For example, the flexible outer layer extends over at least a portion of the connector.

In some implementations, the fluid reservoir extends along the axial direction of the front portion so as to radially cover a first and second section of the front portion, the first section being arranged in front of the second section. Wherein a radial dimension of the fluid reservoir is different at the first section than at the second section. It is also envisaged that two or more fluid reservoirs are formed between the flexible outer layer and the core tube. Preferably, the radial dimension of the fluid reservoir(s) is larger at the first section than at the second section to create a bulb-and-neck shape for sealing against the anal canal of the patient. The fluid reservoir(s) is preferably a single reservoir which extends over the first and second sections, alternatively it is envisaged that the fluid reservoir(s) comprises two separate fluid reservoir(s) units (e.g. a first fluid reservoir forming the bulb and a second fluid reservoir(s) forming the neck). The fluid reservoir(s) at the first and second sections thus forms a radial profile which varies along the elongation axis to form a bulb-and-neck shape with the wide bulb being arranged in front of the narrow neck and configured to enter the anal before the neck.

In some implementations, the fluid reservoir(s) of the second section extends a predetermined distance along the front portion of the core tube and has a constant radius. For example, the predetermined distance may be about 2 to 5 centimeters such that the second section can extend through the entire anal canal when it has been inserted into the anal canal of the patient.

In some implementations, the rear portion comprises an insertion stopper provided on the outside of the rear portion, the insertion stopper having an outer radius larger than the outer radius of the flexible outer layer at the rearward end of the front portion.

With an insertion stopper the catheter is restricted from being inserted too far into the rectum of the patient, thereby mitigating the risk of the patient being injured and ensuring that at least a portion of the rear portion remains outside of the patient after the catheter has been inserted through the anal canal. The insertion stopper may also act as a connector to an external device and/or the flexible outer layer may be attached to the insertion stopper. In some implementations, the fluid reservoir extends rearward up to the insertion stopper, or the fluid reservoir covers at least a front portion of the insertion stopper, softening the impact when the insertion stopper reaches the caudal side of the anal sphincter.

In some implementations, the catheter further comprises a flexible inner layer located between the core tube and the flexible outer layer, wherein the fluid reservoir is formed between the flexible inner layer and the flexible outer layer.

The flexible inner layer is preferably made of the same material as the flexible outer layer. Alternatively, it is envisaged that the flexible inner and outer layers are made from different materials. A benefit with a flexible inner layer is that the outer and inner layer can be made as a single piece, forming an outer sleeve, with an integrated fluid reservoir. Alternatively, the flexible outer layer is attached to the flexible inner layer, e.g. circumferentially attached at two locations thereby forming the annulus fluid reservoir therebetween.

Thus, the fluid reservoir may be filled with a fluid prior to the outer sleeve being pulled over the core tube to form a catheter. For instance, the outer sleeve may be manufactured and/or provided separately from the core tube wherein the catheter is formed by pulling the outer sleeve over the core tube. The flexible inner layer may extend equally far along the elongation axis of the core tube as the flexible outer layer. It is also envisaged that the flexible inner layer ends in front or rearward of the flexible outer layer.

In some implementations, the flexible inner layer has a greater thickness than the flexible outer layer.

With a more rigid flexible inner layer, the outer sleeve may exhibit some degree of structural integrity. For instance, this enables the outer sleeve to form a tubular section which extends in front of and beyond the front portion of the core tube even when the core tube does not comprise a supporting tip portion. Hereby a soft tip is formed. This type of outer sleeve will be comfortable to introduce into the anus of a patient as the flexible outer sleeve will enter the anus first, with the portion of the outer sleeve which is supported by the less flexible core tube trailing behind during insertion.

According to a third aspect of the invention there is provided a method for manufacturing a catheter (such as a rectal catheter). The method comprising the steps of providing a core tube extending along an elongation axis, the core tube comprising a front portion and an opposite rear portion, the core tube further comprising a lumen extending between a front opening in the front portion and a rear opening in the rear portion. The method further comprising arranging a flexible outer layer over the core tube, covering at least partially the front portion in a radial direction and covering the front portion along the elongation axis, the flexible outer layer forming a fluid reservoir between the flexible outer layer and at least the front portion of the core tube. The flexible outer layer further comprises one or more radial eyelet(s) arranged in front of the front portion of the core tube and being in fluid communication with the lumen extending through the core tube, wherein the flexible outer layer is more flexible than the core tube. The method further comprises the step of filling the fluid reservoir with a fluid such that the flexible outer layer and the fluid reservoir forms a resiliently deformable body covering at least a part of the front portion of the core tube.

In some implementations, the flexible outer layer is formed separately and pulled over the core tube. The flexible outer layer may then be attached to the core tube so as to form the fluid reservoir between the core tube and the flexible outer layer wherein the fluid reservoir extends at least partially along the front portion of the core tube. The attachment may be enabled passively, by the flexible outer layer fitting tightly over the core tube at certain locations, or actively by adhering or heat welding the flexible outer layer onto the core tube at certain locations. A fluid is then injected into the fluid reservoir (e.g. by means of a syringe or by allowing the fluid to diffuse through the flexible outer layer). The radial eyelet may be formed after pulling the flexible outer layer over the core tube or, alternatively, the radial outlet is already provided in the flexible outer layer as it is pulled over the core tube.

Additionally, a flexible inner layer may be formed between the flexible outer layer and the core tube.

In some implementations, the method further comprises arranging a flexible inner layer located between the core tube and the flexible outer layer, wherein the fluid reservoir is formed between the flexible inner layer and the flexible outer layer. The flexible inner layer has at least one radial eyelet aligned with the at least one radial outlet of the flexible outer layer wherein the flexible inner layer and the flexible outer layer forms an outer sleeve. Furthermore, arranging the flexible outer layer and flexible inner layers on the core tube may comprise pulling the outer sleeve (comprising the inner and outer flexible layer) over the core tube.

In some implementations, the flexible outer layer is manufactured using a dip-process wherein the core tube is dipped into a fluid material (e.g. silicone) which is then cured over the core tube.

According to a fourth aspect of the invention, there is provided a method for manufacturing a catheter, the method comprising providing a core tube extending from a rear end to a front end along an elongation axis, the core tube comprising a lumen extending between a front opening in a front portion, and a rear opening in a rear portion opposite said front portion. The method further comprises arranging a flexible outer layer over the core tube, the flexible outer layer covering at least partially the front portion in a radial direction, arranging a flexible inner layer between the flexible outer layer and the core tube, wherein the flexible inner layer and the flexible outer layer forms a fluid reservoir therebetween and wherein the flexible outer layer and the flexible inner layer are more flexible than the core tube and further filling the fluid reservoir with a fluid such that the flexible outer layer and the fluid reservoir forms a resiliently deformable body covering at least a part of the front portion of the core tube.

Accordingly, it is entirely optional to form a flexible outer layer which covers the core tube in the elongation axis and in some implementations a flexible inner layer and flexible outer layer are formed wherein neither of these layers necessarily covers the core tube along the elongation axis.

The invention according to the third and fourth aspects features the same or equivalent benefits as the invention according to the first and/or second aspect. Any functions described in relation to a method may have corresponding features in a catheter, and vice versa.

### BRIEF DESCRIPTION OF THE DRAWINGS

The present invention will be described in more detail with reference to the appended drawings, showing currently preferred embodiments of the invention.
Figure 1a is an exterior view of a catheter according to some implementations.
Figure 1b depicts a cross-section of a core tube according to some implementations.
Figure 1c depicts a cross-section of a core tube with a flexible outer layer and fluid reservoir according to some implementations.
Figure 1d depicts a cross-section of the flexible outer layer according to some implementations.
Figure 2a illustrates a core tube according to some implementations.
Figure 2b illustrates a core tube fitted with a first type of tip portion according to some implementations.
Figure 2c illustrates a core tube fitted with a second type of tip portion according to some implementations.
Figure 2d illustrates a cross-sectional view of the second type of tip portion, as seen in along the elongation axis, according to some implementations.
Figure 3a depicts the outer profile of a first type of rear portion, according to some implementations.
Figure 3b depicts the outer profile of a second type of rear portion, according to some implementations.
Figure 3c depicts the outer profile of a third type of rear portion, according to some implementations.
Figure 4a depicts a cross-section of an outer sleeve comprising a flexible outer layer, a flexible inner layer and a fluid reservoir, according to some implementations.
Figure 4b depicts a cross-section of a catheter with an outer sleeve according to some implementations.
Figure 4c depicts a cross-section of a catheter with an outer sleeve which does not cover the core tube along the elongation axis according to some implementations.
Figure 4d depicts a cross-section of an outer sleeve which in some implementations is provided separately from the core tube.
Figure 5a is a flowchart describing a method for manufacturing the catheter.
Figure 5b illustrates a core tube being inserted into a flexible outer layer or outer sleeve, according to some implementations.
Figure 6a is a flowchart describing an alternative method for manufacturing the catheter.
Figure 6b illustrates a core tube being dipped into a fluid material so as to form flexible outer layer or outer sleeve, according to some implementations.

### DETAILED DESCRIPTION OF CURRENTLY PREFERRED EMBODIMENTS

In the following detailed description, preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the catheter, radial extension of the fluid reservoir or flexible outer layer etc. Further, even though the discussed embodiments are in particular suitable for use as rectal irrigation catheters, e.g. for use in Trans Anal Irrigation (TAI) or enema, similar catheters may also be used for other applications, such as for dilation catheters, venous catheters, urinary catheters, and the like.

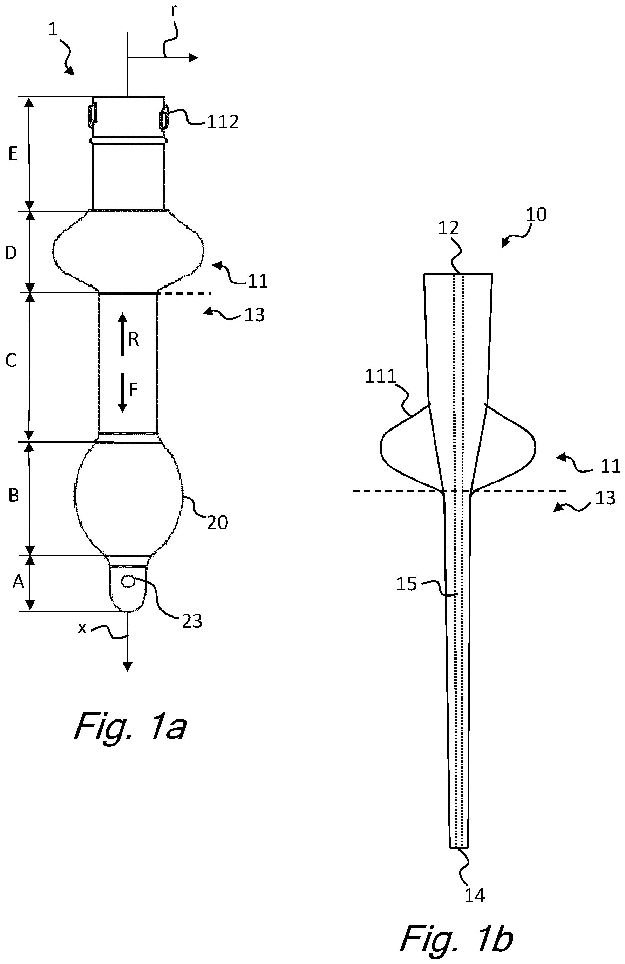

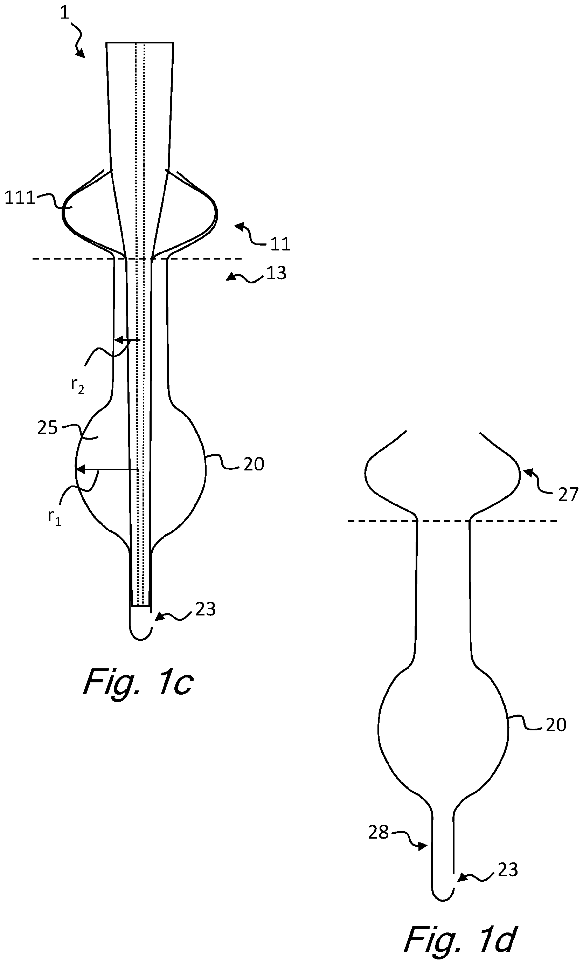

A catheter 1 is depicted schematically in fig. 1a, fig. 1b, fig. 1c, and fig. 1d. The catheter 1 depicted in these figures is a rectal catheter suitable for anal irrigation, e.g. Trans Anal Irrigation (TAI) or enema. It is however envisaged that the catheter 1 also could be used for other purposes, as indicated in the above.

As seen in fig. 1a, the catheter 1 is elongated and extends along an elongation axis x in a forward direction F, being opposite of a rearward direction R. The catheter 1 has a rearward end (in section E) to a front end (in section A). The catheter 1 comprises a core tube 10, and a lumen 15 extending through the core tube 10 to bring a rear opening 12 into fluid communication with a front opening 14. The core tube 10 comprises a front portion 13 (corresponding to sections A-C) which is configured to be at least partially inserted into the anal canal of a patient, and a rear portion 11 (corresponding to sections D and E) which is configured to remain at least partially outside the anal canal of the patient when in use.

The front portion 13 is wrapped with a flexible outer layer 20 (flexible outer sleeve) which in a radial direction r covers at least a portion, or all, of the front portion 13 of the core tube 10 and covers the front portion 13 in the direction of the elongation axis x. The flexible outer layer 20 is provided with a radial eyelet 23 which extends through the flexible outer layer and is in fluid communication with the lumen 15 of the core tube 10. Preferably, the outside front portion 13 is entirely covered by the flexible outer layer 20 in the radial direction r and the direction of the elongation axis x except for at the radial eyelet 23 provided in the flexible outer layer 20.

The flexible outer layer 20 forms a volume between the flexible outer layer 20 and the core tube 10 which surround the core tube 10 for at least a portion of the front portion 13. This volume acts as fluid reservoir 25 configured to be filled with, and retain, a fluid therein. Preferably, the fluid reservoir 25 is in the shape of a toroid circumferentially surrounding the core tube 10. Alternatively, a plurality of separate fluid reservoirs 25 is arranged around the front portion 13 of the core tube 10. For example, the fluid reservoirs 25 are angularly separated while they have the same general shape (e.g. bulb and neck shape). Additionally, or alternatively, the fluid reservoirs 25 are separated in the axial direction.

To ensure that the fluid reservoir 25 is sealed and does not leak any fluid into the environment or into the lumen 15 even when it is subject to deformation (e.g. due to being pressed into the anal canal of a patient) the flexible outer layer 20 may be attached to the core tube 10, e.g. using an adhesive or heat welding, at one or more points along the front portion 13 of the core tube 10. For example, the flexible outer layer may be circumferentially attached to the core tube 10 at a first position in front of the radial eyelet 23 (e.g. in section A or B).

Additionally, or alternatively, the flexible outer layer 20 is attached to the rear portion 11 of the core tube 10 or at a second position located rearward of the first position.

A fluid, such as a gas or a liquid is present in the fluid reservoir 25, meaning that the fluid reservoir 25 becomes flexible and may deform as the flexible outer layer 20 can stretch and relax to allow the fluid to redistribute itself inside the fluid reservoir 25. Preferably, the fluid is an oil, such as a mineral oil. Also, as seen in fig. 1c, the flexible outer layer 20 may be shaped such that, when the fluid reservoir 25 is filled with a fluid, the flexible outer layer assumes a predetermined radial profile along the elongation axis. This inherent radial profile of the flexible outer layer 20 may be any profile. For rectal irrigation purposes, the radial profile preferably comprises bulb-and-neck profile as shown in fig. 1c with a greater outer radius r₁ at a first section (section B) of the front portion 13 and a lesser outer radius r₂ at second section (section C) of the front portion, wherein the first section is arranged in front of the second section. In some implementations, the catheter comprises a third section arranged rearwards of the second section (section D) wherein the third section has an outer radius exceeding the outer radius r₂ of the second segment. This third section may be formed by a protrusion of the core tube 111 (optionally covered with the flexible outer layer 20), a separate fluid reservoir or an extension of the fluid reservoir 25 of the front portion 13.

The rear portion of the core tube 10 may comprise a connector for connecting the catheter 1 to an external device (not shown), such as a fluid tube or a pump system. The connector is configured to allow the external device to come in fluid communication with the lumen 15 of the core tube 10. The connector may comprise threads so as to act as a screw-type connector configured to be mated with corresponding threads on the external device. It is envisaged that any type of connector or fitting may be provided on the rear portion so as to securely attach the catheter 1 to an external device. For instance, the connector may be a bayonet-type connector or fitting which is held together by friction. In some implementations, the flexible outer layer 20 extends at least partially over the connector. In some implementations, the rear portion 11 of the core tube 10 comprises a skirt (not shown) which extends rearwards and surrounds at least a portion of the connector. Preferably, the flexible outer layer 20 extends over the rear portion 11 so as to cover at least a portion of the skirt.

The external device may be an electrically or manually operated pump system which pumps an irrigation fluid from an irrigation fluid reservoir through the catheter 1. For instance, the pump system pumps irrigation fluid from the irrigation fluid reservoir through a flexible tube which is connected to the connector in the rear portion 11 of the catheter 1. Alternatively, the pump system is compact and connected directly to the connector of the catheter 1. In some implementations, the external device comprises a compressible balloon filled with irrigation fluid wherein the user manually squeezes the balloon to inject irrigation fluid through the core tube of the catheter 1.

When the catheter 1 is used, the rear portion 11 is connected to an external device which is configured to pump an irrigation fluid through the lumen of the catheter 1. The catheter 1 is then inserted into the anal canal of the patient with the front portion 13 first. Accordingly, sections A and B are first introduced into the anal canal wherein at least section B comprises the fluid reservoir 25. During insertion, the fluid reservoir 25 is deformed and/or compressed to allow the catheter 1 to pass through the anal canal and the fluid inside the fluid reservoir is at least temporarily displaced. For example, flexible outer layer 25 defines a bulb-and-neck radial profile wherein the front bulb (section B) is first introduced into the anal canal which results in additional fluid being temporarily moved from the bulb to the rear neck portion (section C), trailing behind the bulb portion. Once the bulb has passed the anal sphincter, into the anal canal, the fluid moves back into the bulb section which reinflates and contributes to creating an inside seal against the inside of the anal canal. The catheter 1 is inserted until the entire neck portion, or at least a part of it, is inside the anal canal while the entire bulb section, or at least a part of it, has entered into the rectum.

In some implementations, the rear portion 11 comprises an insertion stopper (section D) comprising a rigid or flexible protrusion which prohibits the catheter 1 from being introduced further into the anal canal. For instance, the insertion stopper is configured to abut the outside of the anal canal when the front portion 13 has been inserted into the anal canal. The insertion stopper may be a protrusion 111 of rear portion 11 of the core tube 10 (as seen in fig. 1b) which optionally is covered with the flexible outer layer 20. Alternatively, it is envisaged that the insertion stopper is flexible and deformable, e.g. formed by the flexible outer layer 20 and a fluid reservoir of the rear portion (not shown). In an exemplary embodiment of this, the fluid reservoir 25 extends to cover also at least a portion of the rear portion 11. Preferably, the insertion stopper has a radius which exceeds that of the most rearward part of the front portion 13. In this way, there will be an increase in the force required to introduce the catheter 1 into the anal canal once the insertion stopper reaches the outside of the anal canal. This increase in force can be felt by the person introducing the catheter 1 into her/his own anal canal, or the anal canal of a patient, and serves as an indicator that the catheter has been properly and fully inserted. While an insertion stopper which is realized with a fluid reservoir extending to cover the rear portion may be more comfortable, a benefit with a less flexible insertion stopper (e.g. formed as a single piece with the core tube 10) is that the catheter will remain inside the anal canal even if the bowels and/or anal canal contracts. If the fluid reservoir extends so as to form part of the insertion stopper, a part of the fluid reservoir will remain outside the anal canal of the patient. If the anal canal suddenly contracts, fluid may be forced into the rear portion which is outside the patient's anal canal, which risks causing the catheter to be forced out of the anal canal of the patient.

With the catheter 1 inserted into the anal canal an irrigation fluid (e.g. water) is injected into the lumen 15 via the rear opening 12 of the core tube. The irrigation fluid exits the lumen 15 via the front opening 14 and then travels through the one or more radial eyelet(s) 23 of the flexible outer layer 20 and enters the anal canal. The deformation of the flexible outer layer 20 and the fluid reservoir 25 caused by the anal canal and anal sphincter creates a fluid tight seal such that no irrigation fluid will leak out via the anal canal until the catheter 1 is removed. After irrigation fluid has been injected, at least a portion (section E) of the rear portion 11 remains outside of the anal canal enabling the catheter 1 to be easily gripped and removed.

In fig. 1d the flexible outer layer 20 is shown removed from the core tube 10. In some implementations the flexible outer layer 20 is formed separately from the core tube 10, e.g. in a mold, thermoformed or in a dipping process with a mandrel, and pulled over the core tube 10 to form the catheter 1. For example, the flexible outer layer 20 is shaped as a cylindrical pouch with a closed front end and an open rear end. The cylindrical pouch having an inner diameter which is equal to or even smaller than the outer diameter of the core tube 10 at a first location 28, rearward of the radial eyelet 23, and at a second location 27 rearward of the first location (e.g. at an insertion stopper protrusion 111 of the core tube). Thus, when the flexible outer layer 20 can be pulled over the core tube 10 the flexible outer layer will be stretched and fitted snuggly around the core tube at the first and second locations 27, 28 separated along the elongation axis x and thus defining an annulus fluid reservoir 25 surrounding at least a part of the front portion 13 of the core tube 10. Additionally, or alternatively, the flexible outer layer 20 is attached (e.g. with means of melting or adhesive) at the two locations 27, 28 to create a fluid tight fluid reservoir 25.

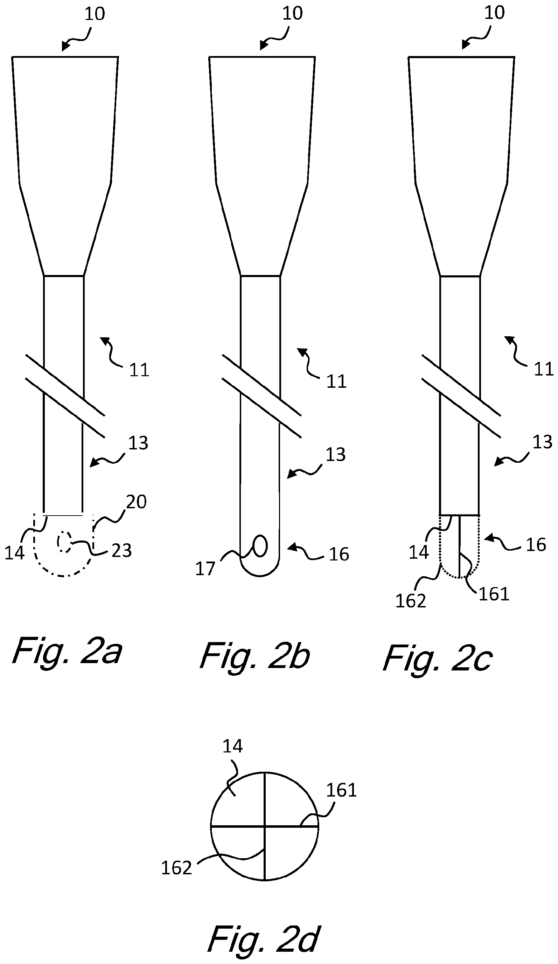

Turning to fig. 2a, fig. 2b, fig. 2c and fig. 2d different examples of tip portions 16 are presented. In some implementations, the core tube 10 comprises a tip portion 16 arranged in front of the front portion 13 and thereby defining the front most tip of the core tube 10.

The tip portion 16 acts as a scaffolding or supporting structure which supports the flexible outer layer 20 along at least one of the radial directions and the elongation axis.

The tip portion 16 in fig. 2b comprises a tubular extension section extending along the direction of the elongation axis, which is terminated with a dome shaped cap at the front end. One or more radial eyelet(s) 17 is provided in the tubular extension section and the one or more radial eyelet(s) 17 in the tubular extension is aligned with the radial eyelet 23 of the flexible outer layer 20 allowing irrigation fluid to pass through the tip portion 16 and out through the radial outlet 23 in the flexible outer layer 20.

The tip portion 16 depicted in fig. 2c and fig. 2d comprises two perpendicular planar elements 161, 162 (forming a cross as seen along the direction of the elongation axis in fig. 2d) wherein a fluid being ejected from the front opening 14 passes the planar elements in one of four quadrants and exits through the one or more radial eyelet(s) 23 in the flexible outer layer 20. It is preferable that the outer surface of the front end of the tip portion 16 is rounded so as to ensure comfortable introduction of the tip portion 16 into the anal canal. To this end, the front most part of the two perpendicular planar elements 161, 162 has rounded edges which support the flexible outer layer. It is also envisaged that a dome shaped cap could be attached onto the two perpendicular planar elements 161, 162 to enable a rounded front most portion while still allowing irrigation fluid to exit through the radial eyelet 23 via the four quadrants.

The different tip portions 16 illustrated in fig. 2b, fig. 2c, and fig. 2d are merely exemplary and many more (not shown) variants of tip portions 16 are also envisaged. For instance, a single planar element 161 or more than two planar elements can be used while still achieving the effect of supporting the flexible outer layer 20 along at least the elongation axis and allowing irrigation fluid to pass from the front opening 14 to the radial eyelet 23 in the flexible outer layer 20.

The tip portion 16 is optional, and in some embodiments the core tube does not comprise a tip portion 16 which supports the flexible outer layer along the elongation axis. The flexible outer layer, while being flexible and/or elastic may feature some degree of structural integrity, meaning that the flexible outer layer supports itself so as to e.g. assume a cylindrical shape with a dome, bulbous or convex front tip surface (as seen in fig. 2a). Nevertheless, and even if the flexible outer layer is a thin sheet having a low degree of, or essentially no, structural integrity, the flexible outer layer 20 arranged in front of the front opening 14 in the core tube 10 will inflate and inject fluid through the radial eyelet 23 once the irrigation fluid is injected through the lumen in the core tube 10.

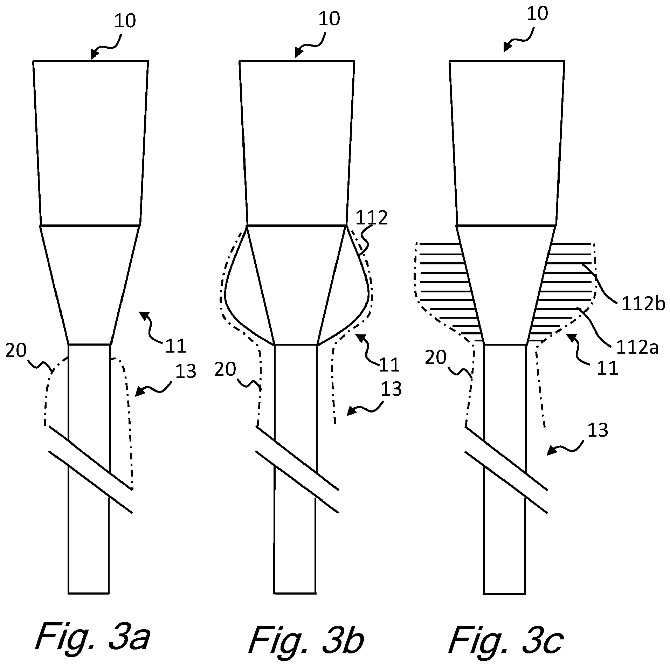

Fig. 3a, fig. 3b and fig. 3c depict exemplary embodiments of how the flexible outer layer 20 can be attached to the core tube 10.

As seen in fig. 3a, the flexible outer layer 20 may be attached at a location along the front portion 13. For instance, the flexible outer layer 20 is circumferentially attached to the outside of the front portion 13 of the core tube 10 using an adhesive or heat welding.

In fig. 3b, the flexible outer layer 20 is pulled over a radial protrusion 112 on the core tube (e.g. also acting as an insertion stopper) and retained on the core tube 10 by the radial protrusion 112 by stretching the flexible outer layer 20. In some implementations, the flexible outer layer 20 is formed as a single piece (e.g. using a mold) and pulled over the core tube 10. The flexible outer layer 20 may be manufactured with a radial profile having a protrusion corresponding to the protrusion 112 of the core tube such that when the flexible outer layer is pulled over the core tube 10 the flexible outer layer fits tightly around the radial protrusion 112. Additionally, the flexible outer layer may also be heat welded or adhered to the radial protrusion.

In one embodiment shown in fig. 3c, the rear portion 11 comprises a plurality of radial protrusions 112a, 112b (e.g. also acting as an insertion stopper) over which the flexible outer layer 20 is pulled so as to retain the flexible outer layer 20 on the core tube 10. The radial protrusions 112a, 112b are spaced apart at regular or intermittent separation distances. The radial protrusions 112a, 112b may have a gradually increasing radial displacement in the front to rear direction. The radial profile of the radial protrusions may be that of a disc, polygon or any other shape.

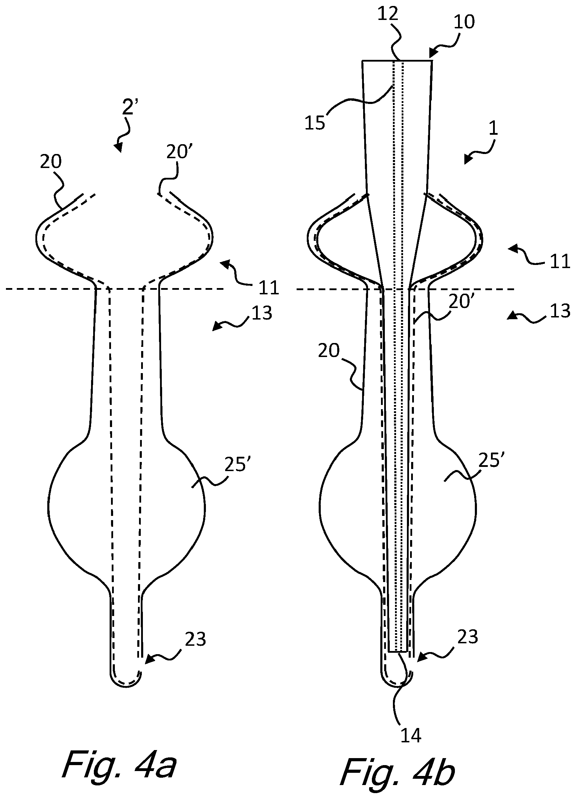

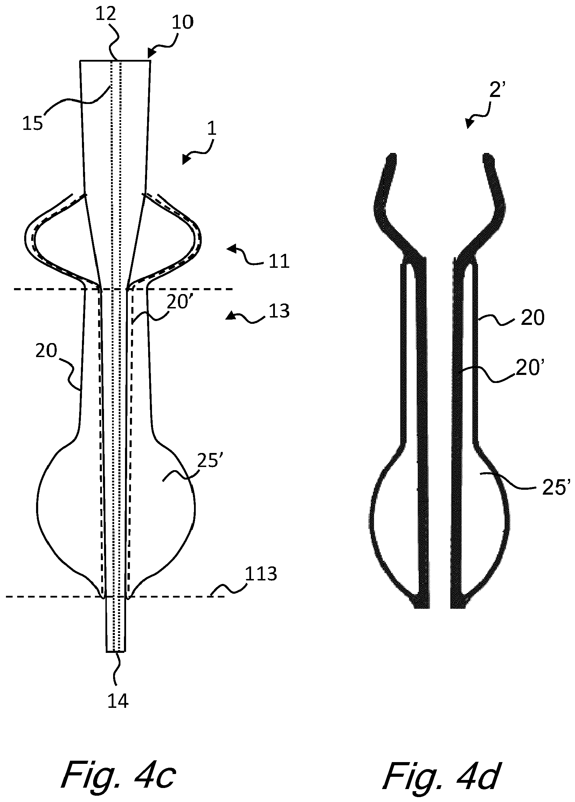

In some implementations, the catheter 1 further comprises a flexible inner layer 20' in addition to the flexible outer layer as shown in fig. 4a, fig. 4b, fig. 4c and fig. 4d. The flexible inner layer 20' may be made of the same material as the flexible outer layer 20, such as silicone, or it is envisaged that the flexible inner layer is made of a different material. The flexible inner and outer layers 20, 20' may have the same thickness, or it is envisaged that the inner and outer layer 20, 20' have different thicknesses (e.g. the flexible inner layer 20' having a greater thickness than the flexible outer layer 20).

At some locations along the elongation axis, of the core tube the flexible inner and outer layers 20, 20' are abutting and/or attached to each other. For example, in fig. 4 a and fig 4b, the flexible inner layer 20' comprises a radial eyelet aligned with radial eyelet 23 of the flexible outer layer and the flexible inner and outer layers may be abutting and/or attached to each other at the rear portion 11 or at a location rearward of the radial eyelet 23 and in front of the fluid reservoir 25'. However, at the fluid reservoir 25' located around at least a part of the front portion 13 of the catheter, the flexible inner and outer layer 20, 20' are separated by the fluid in the fluid reservoir 25'. Thus, the fluid reservoir 25' may be defined by a cavity between the inner and flexible outer layer 20, 20'.

As the flexible outer layer 20 undergoes deformation and allows the fluid in the fluid reservoir 25' to be redistributed it is preferable that the flexible outer layer 20 is thin, flexible and/or elastic. The flexible inner layer 20' may be equally thin and/or elastic although the flexible inner layer 20', being closer and abutting the less flexible core tube 10 to a greater extent than the flexible outer layer 20, is not expected to be deformed to the same extent as the flexible outer layer 20. In some implementations, the flexible inner and outer layer 20, 20' will be abutting and extend beyond and in front the core tube 10 as shown in fig. 4b when the core tube 10 is not provided with a tip portion. In these and other implementations it is preferable if the flexible inner layer 20' is thicker and/or more rigid than the flexible outer layer 20 such that the front most portion of the flexible inner and outer layer 20, 20' is supported by at least the rigidity of the flexible inner layer 20'.

In some implementations, the flexible inner layer 20' abuts the core tube 10 along the entire length of the front portion 13. Additionally, it is understood that any embodiment comprising a flexible inner layer 20' in addition to the flexible outer layer 20 may be used with any of the tip portions described in relation to fig. 2b-2d or any of the rear portions 11 described in relation to fig. 3b and fig. 3c. Furthermore, the flexible inner and/or outer layer 20, 20' may also be attached to some location of the front portion 13 as shown in fig. 3a. For example, the flexible inner layer 20' may be circumferentially attached to the outside of the front portion 13 of the core tube 10 at a given location with the flexible outer layer being attached to the flexible inner layer or the core tube 10 in front of, rearward of or at the same location as the given location. Additionally or alternatively, the flexible outer layer may be attached to the flexible inner layer at two or more, or three or more locations such as along two or more axially or radially separated connection lines. In one example the flexible outer layer is attached to the flexible inner layer at three or more locations so as to form two or more separate fluid reservoirs.

In fig. 4c and 4d a catheter 1 and outer sleeve 2' according to some embodiments is shown. As seen in fig. 4c, embodiments are envisaged in which the flexible outer layer 20 (and flexible inner layer 20') does not extend so as to cover the core tube 10 along the elongation axis. For instance, the core tube 10 extends beyond the flexible inner layer 20 and flexible outer layer 20', ends in line with flexible inner layer 20 and flexible outer layer 20' (not shown) or ends rearwards of the front most part of flexible inner layer 20 and flexible outer layer 20' (not shown). While implementations with a flexible outer layer 20 (and flexible inner layer 20') covering the core tube 10 along the elongation axis inherently prohibits the flexible outer layer 20 (and flexible inner layer 20') it is envisaged that the flexible outer layer 20 (and flexible inner layer 20') could be retained on the core tubes 10 by other means, e.g. at fixation location 113. In one exemplary embodiment, the flexible inner layer 20' and flexible outer layer 20 is fused or adhered to the core tube 10 at a fixation position 113 along the front portion 13. Alternatively or additionally, the flexible inner layer 20' and flexible outer layer 20 may be shaped so as to fit tightly around the core tube 10 wherein the flexible inner layer 20' and flexible outer layer 20 are retained by friction.

In fig. 4d there is illustrated how the flexible inner layer 20' and flexible outer layer 20 may form an outer sleeve 2' which e.g. is provided as a separate piece with or without a fluid provided in the fluid reservoir 25'.

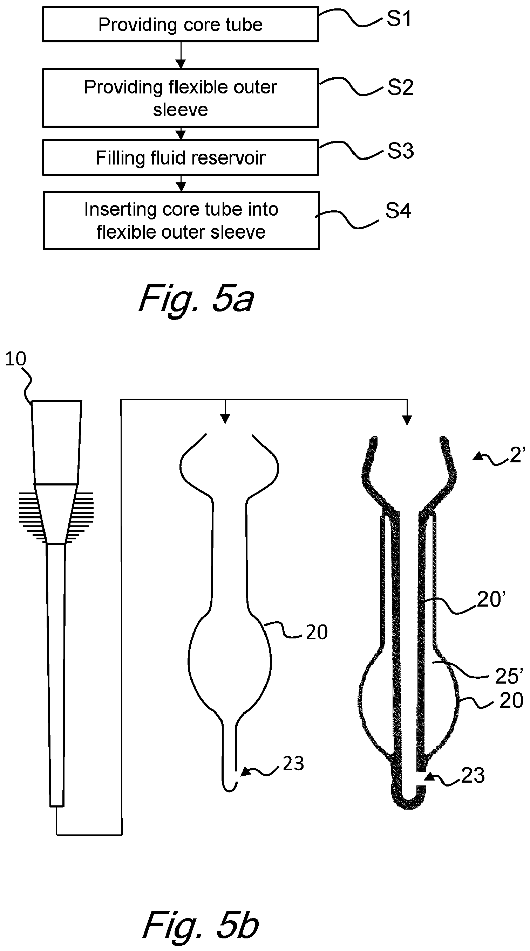

Fig. 5a is a flowchart illustrating a method for manufacturing the catheter. With further reference to fig. 5b, a core tube 10 is proved at step S1. For example, the core tube 10 comprises a lumen for transporting irrigation fluid through the core tube 10. At step S2, a flexible outer sleeve 2' having a fluid reservoir 25' disposed between the flexible outer layer 20 and the flexible inner layer 20' is provided. For instance, the flexible outer sleeve 2' has been manufactured separately using e.g. a mold process, thermoforming process or dipping process wherein a mandrel is dipped into a fluid material which is cured and removed from the mandrel. Then, at step S3 the fluid is added to the reservoir 25' of the flexible outer sleeve 2'. Adding the fluid may comprise injecting the fluid with a syringe, allowing the fluid to diffuse through the flexible inner layer 20 and/or flexible outer layer 20' or adding the fluid before sealing the fluid reservoir 25'. At step S4, the core tube 10 is inserted into the flexible outer sleeve 2'. Optionally, step S4 may further comprise attaching the flexible outer sleeve 2' to the core tube 10 at one or more locations so as to retain the flexible outer sleeve 2' on the core tube 10. In some implementations, the flexible outer sleeve 2' fits tightly over core tube 10 so as to thereby retain the flexible outer sleeve 2'. It is envisaged that the order of some of the steps in fig. 5a may different, e.g. it is envisaged that step S4 is performed prior to step S3 meaning that the fluid is added to the fluid reservoir 25' after the core tube 10 has been inserted into the outer sleeve 2'.

While fig. 5b depicts a flexible outer layer 20 and an outer sleeve 2' which covers the core tube along the elongation axis it is understood that the same method may be used to manufacture a catheter with an outer sleeve 2' which does not cover the core tube 10 along the elongation axis (e.g. allowing the core tube 10 to pass through as seen in fig. 4c).

In the above, a method for manufacturing a catheter with a flexible outer sleeve 2' comprising an inner flexible layer 20', an outer flexible layer 20 and a fluid reservoir 25' disposed there between is described. It is however also envisaged that a catheter with only a flexible outer layer 20 (and no flexible inner layer 20') can be formed using a similar process. As an example of such a manufacturing process step S2 consists of providing a flexible outer layer 20. For instance, the flexible outer layer has been formed separately, e.g. using a mold and/or mandrel process. Then, at a first alternative step following step S2 the core tube 10 is inserted into the flexible outer layer 20. Optionally, the first alternative step comprises attaching the flexible outer layer 20 to the core tube at two or more locations so as to form a fluid reservoir between the flexible outer layer 20 and the core tube 10. At a second alternative step, a fluid is added to the fluid reservoir between the flexible outer layer and the core tube 10. This may be achieved by injecting the fluid with a syringe, allowing the fluid to diffuse through the flexible outer layer 20, or adding the fluid before sealing the fluid reservoir against the core tube 10.

Aligned eyelet(s) of the flexible outer layer, and optionally of the flexible inner layer, may be formed at any time in the manufacturing process, e.g. after or before the outer sleeve 2' (or flexible outer layer 20) has been arranged on the core tube 10 and/or after or before the fluid reservoir 25' has been filled with a fluid.

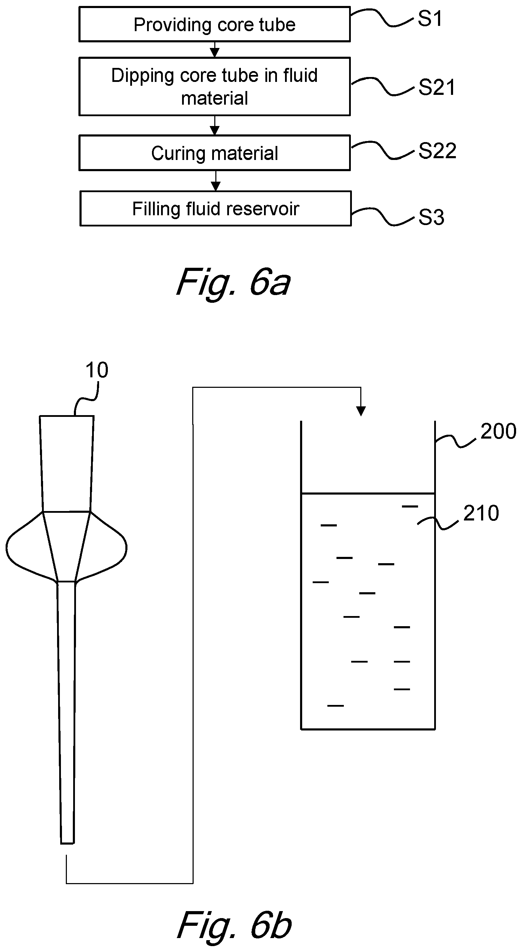

Fig. 6a and fig. 6b illustrates an alternative method for manufacturing a catheter. At step S1, a core tube 10 is provided and at steps S21 and S22 the core tube 10 is repeatedly dipped in a tank 200 of fluid material 210 (e.g. silicone). By controlling the temperature of the fluid material 210, the speed at which the core tube 10 is inserted into the fluid material 210, and the time during which the core tube 10, remains inserted into the fluid material 210, the flexible outer layer 20 can be formed onto the core tube 10. Using the same technique, a flexible outer sleeve can also be formed onto the core tube. Then, at step S3, the fluid reservoir is filled with a fluid. To prohibit the fluid material from entering into the lumen of the core tube 10 a solvable material may be used to close the lumen when the core tube 10 is submerged into the fluid material. Subsequently, the solvable material may be removed by flushing a solvent through the lumen.

Aligned eyelet(s) of the flexible outer layer, and optionally of the flexible inner layer, may be formed at any time in the manufacturing process, e.g. after or before the fluid reservoir has been filled.

Optionally, instead of performing the dipping process with the core tube 10 directly, it is envisaged that a mandrel could be used to form the flexible outer layer or the outer sleeve whereby the flexible outer layer or outer sleeve is then removed from the mandrel and pulled over the core tube 10 as described in the above. Thus, steps S21 and S22 of fig. 6a may be comprised in step S2 of fig. 5a which involves providing a flexible outer layer 20 or flexible outer sleeve 2'.

Suitable methods to form the outer sleeve is for example described in US application number 15/281,232 and US patent number 6,626,888, 6,479,000 and 5,981,954, all of the documents hereby incorporated in their entirety by reference.
The person skilled in the art realizes that the present invention by no means is limited to the preferred embodiments described above. On the contrary, many modifications and variations are possible within the scope of the appended claims. For example, while the front portion is at least partially covered with a surrounding fluid reservoir it is envisaged that the fluid reservoir does not extend over a predetermined portion of the front portion. As seen e.g. in fig. 1c and fig. 4b the flexible outer layer 20 (and optionally the flexible inner layer 20') lies in direct contact with the core tube 10 at a portion in front of the fluid reservoir 25 but rearward of the front opening 14 and the radial eyelet 23. In the claims, the word "comprising" does not exclude the presence of other elements or steps than those listed in the claim. The word "a" or "an" preceding an element does not exclude the presence of a plurality of such elements.

## Claims

1. A catheter (1) comprising:
a core tube (10) extending from a rear end to a front end along an elongation axis (x), the core tube (10) comprising a lumen (15) extending between a front opening (14) in a front portion (13) and a rear opening (12) in a rear portion (11), opposite said front portion (13),
a flexible outer layer (20) disposed around the front portion (13), the flexible outer layer (20) covering the front portion (13) along the elongation axis (x) and at least partially covering the front portion (13) in a radial direction (r), the flexible outer layer (20) forming a fluid reservoir (25, 25') containing a fluid between the flexible outer layer (20) and at least a part of the front portion (13), such that the flexible outer layer (20) and the fluid reservoir (25, 25') forms a resiliently deformable body covering at least a part of the front portion (13) of the core tube (10),
wherein the flexible outer layer (20) further comprises one or more radial eyelet(s) (23) arranged in front of the front portion (13) of the core tube (10) and being in fluid communication with the front opening (14), and
wherein the flexible outer layer (20) is more flexible than the core tube (10).

2. A catheter (1) comprising:
a core tube (10) extending from a rear end to a front end along an elongation axis (x), the core tube (10) comprising a lumen (15) extending between a front opening (14) in a front portion (13) and a rear opening (12) in a rear portion (11), opposite said front portion (13),
a flexible outer layer (20) disposed around the front portion (13), the flexible outer layer (20) at least partially covering the front portion (13) in a radial direction (r),
a flexible inner layer (20') disposed between the flexible outer layer (20) and the core tube (10),
wherein the flexible outer layer (20) and the flexible inner layer (20') forms a fluid reservoir (25') containing a fluid between the flexible outer layer (20) and the flexible inner layer (20'), such that the flexible outer layer (20) and the fluid reservoir (25') forms a resiliently deformable body covering at least a part of the front portion (13) of the core tube (10),
wherein the flexible outer layer (20) and flexible inner layer (20') are more flexible than the core tube (10).

3. The catheter (1) according to claim 1 or claim 2, wherein the core tube (10) further comprises a tip portion (16) arranged at the front end of the front portion (13), wherein the tip portion (16) supports the flexible outer layer (20) along the elongation axis (x).

4. The catheter (1) according to claim 3 when depending on claim 1, wherein the tip portion (16) comprises at least one radial opening (17) in fluid communication with the lumen (15) and the one or more radial eyelet(s) (23) of the flexible outer layer (20).

5. The catheter (1) according to any of the preceding claims, wherein the flexible outer layer (20) is attached to at least a part of the outside of the rear portion (11).

6. The catheter (1) according to claim 5, wherein the part of the outside of the rear portion (11) to which the flexible outer layer (20) is attached is provided with at least one radial protrusion (112, 112a, 112b) configured to hold the flexible outer layer (20) in place using friction.

7. The catheter (1) according to any of the preceding claims, wherein said rear portion (11) further comprises a connector for connecting the rear opening (12) to an external device.

8. The catheter (1) according to any of the preceding claims, wherein the fluid reservoir(s) (25, 25') extends along the axial direction (x) of the front portion (13) so as to radially cover a first and second section (B, C) of the front portion (13), the first section (B) being arranged in front of the second section (C),
wherein a radial dimension of the fluid reservoir (25, 25') is different at the first section (B) than at the second section (C).

9. The catheter (1) according to any of the preceding claims, wherein the rear portion (11) comprises an insertion stopper (112, 112a, 112b) provided on the outside of the rear portion (11), the insertion stopper (112, 112a, 112b) having an outer radius larger than the outer radius of the flexible outer layer (20) at a rear end of the front portion (13).

10. The catheter (1) according to claim 1, further comprising:
a flexible inner layer (20') located between the core tube (10) and the flexible outer layer (20), wherein said fluid reservoir (25') is formed between said flexible inner layer (20') and said flexible outer layer (20).

11. The catheter (1) according to any of claims 2-10, wherein the flexible inner layer (20') has a greater thickness than the flexible outer layer (20).

12. The catheter (1) according to any of the preceding claims, wherein the catheter (1) is a rectal catheter

13. A method for manufacturing a catheter (1), the method comprising:
providing (S1) a core tube (10) extending from a rear end to a front end along an elongation axis (x), the core tube (10) comprising a lumen (15) extending between a front opening (14) in a front portion (13), and a rear opening (12) in a rear portion (11) opposite said front portion (13);
arranging (S21, S22, S4) a flexible outer layer (20) over the core tube (10), covering at least partially the front portion (13) in a radial direction (r) and covering the front portion (13) along the elongation axis (x), the flexible outer layer (20) forming a fluid reservoir (25, 25') between the flexible outer layer (20) and at least the front portion (13) of the core tube (10), wherein the flexible outer layer (20) further comprises one or more radial eyelet(s) (23) arranged in front of the front portion (13) of the core tube (10) and being in fluid communication with the front opening (14) of said lumen (15), wherein the flexible outer layer (20) is more flexible than the core tube (10); and
filling (S3) the fluid reservoir (25, 25') with a fluid such that the flexible outer layer (20) and the fluid reservoir (25, 25') forms a resiliently deformable body covering at least a part of the front portion (13) of the core tube (10).

14. The method of claim 13, further comprising:
arranging a flexible inner layer (20') located between the core tube (10) and the flexible outer layer (20), wherein said fluid reservoir (25') is formed between said flexible inner layer (20') and said flexible outer layer (20) and the flexible inner layer (20') has at least one radial eyelet aligned with the at least one radial outlet (23) of the flexible outer layer (20), wherein said flexible inner layer (20') and said flexible outer layer (20) forms an outer sleeve (2'); wherein
arranging (S4) the flexible outer layer (20) and flexible inner layer (20') on the core tube (10) comprises pulling the outer sleeve (2') over the core tube (10).

15. A method for manufacturing a catheter (1), the method comprising:
providing (S1) a core tube (10) extending from a rear end to a front end along an elongation axis (x), the core tube (10) comprising a lumen (15) extending between a front opening (14) in a front portion (13), and a rear opening (12) in a rear portion (11) opposite said front portion (13);
arranging (S21, S22, S4) a flexible outer layer (20) over the core tube (10), the flexible outer layer (20) covering at least partially the front portion (13) in a radial direction (r),
arranging a flexible inner layer (20') between the flexible outer layer (20) and the core tube (10), wherein the flexible inner layer (20') and the flexible outer layer (20) forms a fluid reservoir (25') therebetween and wherein the flexible outer layer (20) and the flexible inner layer (20') are more flexible than the core tube (10); and
filling (S3) the fluid reservoir (25') with a fluid such that the flexible outer layer (20) and the fluid reservoir (25') forms a resiliently deformable body covering at least a part of the front portion (13) of the core tube (10).
